# EUROPEAN PATENT SPECIFICATION

(11) **EP 2 736 481 B1**
(45) Date of publication and mention of the grant of the patent: **19.07.2017**
(21) Application number: 12753197.8
(22) Date of filing: 27.07.2012
(51) Int. Cl.: A61K 8/22, A61K 8/46, A61K 8/67, A61Q 5/08

(54) **METHOD OF REMOVING COLOUR FROM OXIDATIVELY DYED HAIR**
VERFAHREN ZUR ENTFERNUNG VON FARBE AUS OXIDATIV GEFÄRBTEM HAAR
PROCÉDÉ D'ÉLIMINATION D'UNE COLORATION OXYDATIVE DE LA CHEVELURE

(30) Priority: 29.07.2011 GB 201113087
(43) Date of publication of application: 04.06.2014
(73) Proprietor: Perachem Limited, Leeds, Yorkshire LS19 7XP (GB)
(72) Inventor: HAWKES, Jamie Anthony, Leeds Yorkshire LS20 9PS (GB); LEWIS, David Malcolm, Otley LS21 2AL (GB); MAMA, John, Leeds LS8 2JB (GB)
(74) Representative: Appleyard Lees IP LLP
(86) International application number: PCT/GB2012/051839
(87) International publication number: WO 2013/017862

(56) References cited:
- FR-A1- 2 657 781
- US-A1- 2008 085 249
- US-B1- 6 379 657
- A. KUZUHARA: "Influence of urea on the coloring ability of a low-temperature coloring method of keratin fibers using polyethyleneimine", JOURNAL OF APPLIED POLYMER SCIENCE, vol. 91, no. 6, 1 January 2004 (2004-01-01), pages 3827-3834, XP055072994, ISSN: 0021-8995, DOI: 10.1002/app.13582

## Description

The present invention relates to a method of removing colour from dyed hair. In particular it relates to a method for removing colour from hair that has been oxidatively dyed.

The present applicant has developed excellent new compositions for use in colouring hair. These are based on thioether containing compounds or on a combination of a sulfur-containing nucleophile and a dye compound other than a reactive dye; and are described in WO 2010/032034 and WO 2009/112858. One advantage of these hair colouring compositions is that they can also be easily removed by application of a suitable colour removal composition following which the hair is returned to its original colour.

However many hair colouring compositions currently in use are based on oxidative dyes. Oxidative dyes use a combination of aromatic species to colour the hair. Current methods for removing oxidative dyes from the hair are not very reliable and often involve the use of strong bleaching agents. This can be damaging to the hair and an orange tone is often observed i.e. the hair is not returned to its original colour. Other commercially available compositions involve the use of formaldehyde or compounds which may produce formaldehyde in use. Formaldehyde is a known carcinogen and its use in cosmetic applications is strictly limited.

It is an aim of the present invention to provide an improved method of removing colour from hair which has been oxidatively dyed. In particular it is desired that the method does not remove natural pigment from the hair and causes very little or no damage.

According to a first aspect of the present invention there is provided a method of removing colour from hair that has been oxidatively dyed, the method comprising the steps of:
(a) contacting the hair with a composition comprising formamidine sulfinic acid;
(b) contacting the hair with an acidic composition; and
(c) contacting the hair with an oxidising composition;
wherein there is no rinsing step between step (a) and step (b).

By hair that has been oxidatively dyed, we mean to refer to hair that has been treated with an oxidative hair dye. Oxidative hair dyes are well known and the skilled person will recognise the types of compounds used. It will be appreciated that the precise nature of the coloured species present in the hair is often not clear since they are formed *in situ* by treatment of the hair with one or more precursor compositions (suitably containing an aromatic amine and a coupler compound) and an oxidising composition. Typically when hair is oxidatively dyed an aromatic amine composition and a coupler are mixed with a developer immediately before application to the hair. The developer is usually an oxidising composition containing for example hydrogen peroxide. The aromatic amine and coupler precursor compositions comprise small aromatic compounds which interact in the presence of the oxidising developer to form large aromatic conjugated species which are coloured. Although these compounds are referred to as "hair dyes", the resulting coloured species may more correctly be referred to as pigments since they are usually water insoluble.

Aromatic amine and coupler precursor compounds used in the preparation of oxidiative hair dyes are small aromatic compounds, for example benzene or heterocyclic moieties which are substituted with one or more substituents. Suitable substitutents include amino, hydroxy, chloro, alkyl (especially methyl), alkoxy (especially methoxy or ethoxy) and hydroxyalkyl amino (especially di(hydroxyethylene)amino) and hydroxy ethyleneamino).

Suitable oxidative hair dye precursor compositions typically contain at least two small aromatic species, preferably based on benzene. Preferably at least one of the precursor compounds includes at least one amino substituent. Preferred precursor compounds are di or tri substituted.

The resulting coloured species present in hair that has been oxidatively dyed often contain azine residues and may be represented "Ar-N=Ar=N-Ar-N...".
Step (a) of the method of the present invention involves contacting the hair with a composition comprising formamidine sulfinic acid. The colour removal composition comprises formamidine sulfinic acid. Formamidine sulfinic acid is a sulfur-containing nucleophile precursor.

Preferably the composition used in step (a) of the method of the present invention comprises formamidine sulphinic acid. This may also be provided as the tautomer, thiourea dioxide. Thiourea dioxide is not nucleophilic in itself but rearranges to form formamidine sulfinic acid which hydrolyses to form the nucleophilic species HS02 (hydrosulfoxylate). Under acidic conditions formamidine sulphinic acid exists as the thiourea dioxide tautomer but under mildly alkaline conditions the formamidine tautomer is formed which hydrolyses to release HSO₂^{- +}M which is believed to be the active colour removal species. It is also possible to use mixed formamidine / carbamoyl sulphinic acids to generate the reactive species.

It is known from the prior art to use aminomethanesulfinic acid for colour removal. Under the conditions of use this compound was not very effective but for the avoidance of doubt, the compositions used in step (a) of the present invention do not comprise aminomethanesulfinic acid.

The composition used in step (a) preferably comprises at least 0.1 wt% of the sulfur-containing nucleophile or precursor thereof, preferably at least 0.5 wt%, more preferably at least 1 wt%, preferably at least 2 wt%, suitably at least 3 wt%, for example at least 5 wt%.

Suitably the composition used in step (a) comprises up to 40 wt% of the sulfur-containing nucleophile or precursor thereof, preferably up to 30 wt%, more preferably up to 20 wt%, preferably up to 15 wt%, more preferably up to 10 wt%, suitably up to 9 wt%, for example up to 8 wt%, or up to 7 wt%.

The composition used in step (a) may comprise a mixture of two or more sulfur-containing nucleophiles or precursors thereof. The above amounts refer to the total amount of all sulfur-containing nucleophiles or precursors thereof present in the composition.

In some especially preferred embodiments the composition used in step (a) comprises thioglycolic acid and formamidine sulfinic acid.

The composition used in step (a) preferably comprises 0.01 to 10 wt% thioglycolic acid, preferably 0.1 to 5 wt%, more preferably 0.25 to 2 wt%.

The composition used in step (a) preferably comprises 0.1 to 20 wt% formamidine sulfinic acid, preferably 1 to 10 wt%, more preferably 2.5 to 7.5 wt%.

The composition used in step (a) may further comprise one or more of a swelling agent, a surfactant, an activator, a diluent, a conditioning agent, a pH buffer and a thickener.

Other optional excipients may also be present. Preferred ingredients for use as a conditioning agent and other excipients may be selected from those specified on the INCI list(International Nomenclature of Cosmetic Ingredients list). This is drawn up by the Scientific Committee on Consumer Products (SCCP) managed by the Directorate-General for Health and Consumer Protection of the European Commission. The SCCP approve a list of chemicals for use in cosmetics which is referred to as the INCI list.

Suitable swelling agents include urea.

The composition used in step (a) preferably comprises at least 0.1 wt% urea, preferably at least 1 wt%, more preferably at least 2 wt%, most preferably at least 3 wt%.

In some preferred embodiments the composition comprises at least 5 wt% urea, suitably at least 10 wt%, for example at least 12 wt% or at least 15 wt%.

In some embodiments the composition used in step (a) comprises up to 30 wt% urea, preferably up to 20 wt%, for example up to 10 wt% and of up to 7.5 wt%.

In some preferred embodiments the composition comprises up to 50 wt% urea, preferably up to 40 wt% urea, suitably up to 30 wt% urea, for example up to 25 wt% or up to 22 wt%.

In preferred embodiments the compositions used in step (a) comprises a surfactant. Suitable surfactants include anionic, cationic, amphoteric and non-ionic surfactants. A mixture of surfactants may be present. Preferably the composition used in step (a) comprises at least 0.1 wt% of one or more surfactants, preferably at least 0.5 wt%, more preferably at least 1 wt%, for example at least 2 wt% or at least 3 wt%.

The composition used in step (a) suitably comprises up to 40 wt% of one or more surfactants, preferably up to 30 wt%, more preferably up to 20 wt%, suitably up to 15 wt%, for example up to 10 wt% or up to 7 wt%.

In preferred embodiments the composition used in step (a) comprises a non-ionic surfactant, suitably in an amount of from 0.1 to 20 wt%, preferably from 1 to 10 wt%.

Preferred non-ionic surfactants for use herein include alkoxylated non-ionic surfactants. Compounds of this type are typically esters and ethers including an alkyl or alkenyl chain of from 6 to 36 carbon atoms and two or more ethoxy and/or propoxy residues.

Especially preferred non-ionic surfactants are ethoxylated non-ionic surfactants. Examples include ethoxylated sorbitan esters, for example those known as polysorbate 20 and polysorbate 80.

Suitable activators for inclusion in the composition used in step (a) include divalent and trivalent metal species, for example divalent and/or trivalent ions of zinc, magnesium, aluminium and calcium. Preferably the activator includes zinc or especially magnesium ions. The divalent or trivalent ions may be provided in any suitable form. Preferably they are provided as salts, for example carbonates, sulfates, chlorides, acetates or formates. More preferably they are provided as organic acid salts, for example formate or acetate. Suitably the activator may be selected from zinc acetate, magnesium acetate, zinc oxide, magnesium carbonate, zinc sulphate, aluminium acetate, calcium acetate and mixtures thereof. Zinc acetate and magnesium acetate are particularly preferred.

Acetate or formate salts of divalent or trivalent metals when present may be used directly in the compositions used in step (a) of the present invention. Alternatively the corresponding acid and a different metal salt may be used, for example magnesium sulphate and acetic acid.

The composition used in step (a) preferably comprises at least 0.1 wt% of one or more activators, preferably at least 0.5 wt%, more preferably at least 1 wt%, preferably at least 2 wt%, more preferably at least 3 wt%, for example at least 4 wt%.

Suitably the composition used I step (a) comprises up to 30 wt% of one or more activators, preferably up to 20 wt%, more preferably up to 15 wt%, preferably up to 12 wt%, suitably up to 10 wt%, preferably up to 7.5 wt%, for example up to 6 wt%.

Suitable pH buffers include 2-amino-2-methyl-1-propanol. Other suitable buffers will be known to the person skilled in the art.

Suitably the composition used in step (a) has a pH of from 3 to 11. Preferably the composition has a pH of from 7 to 10, for example from 9 to 10 but a lower pH is still practical.

A preferred thickener is hydroxyethylcellulose. Other suitable thickeners will be known to the person skilled in the art. Preferably the composition used in step (a) comprises from 0.1 to 20 wt%, more preferably from 1 to 5 wt% of thickeners.

The composition used in step (a) is preferably an aqueous composition. It preferably comprises at least 40 wt% water, preferably at least 50 wt%, preferably at least 60 wt%, for example at least 70 wt%. It may comprise up to 98 wt% water, suitably up to 95 wt% or up to 90 wt%.

In order to maximise the shelf life of the composition used in step (a) it can be packaged as a multi-component system, the parts of which may be mixed together shortly before use. Such a multi-component product may be packaged as two or more separate precursor compositions which may be provided as powders or granular compositions, liquids or gels, pastes or creams. The viscosity of the precursor compositions may change significantly on admixture.

In embodiments in which the composition is packaged as a multi component system the method of the first aspect of the present invention suitably involves a step prior to step (a) of preparing a composition comprising a sulfur-containing nucleophile or a precursor thereof.

In step (a) of the method of the present invention, the composition is suitably applied to the hair and maintained on the head at a temperature of from 10 to 75 ºC, preferably from 20 to 60 ºC, more preferably from 30 to 50 ºC.

When using temperatures above ambient temperature a suitable hood can be employed to achieve the required temperature.

Suitably the composition is contacted with the hair in step (a) for a period of at least 30 seconds, preferably at least 1 minute, for example at least 5 minutes. In step (a) the composition may be contacted with the hair for a period of up to 2 hours, suitably up to 1.5 hours, for example up to 1 hour. The time for which the composition should be left in contact with the hair will depend on the starting colour of the hair. For hair that has been dyed with a light colour, colour removal can usually be achieved within 10 to 20 minutes. For black hair up to 60 minutes may be needed. The extent of the colour removal can be monitored by visual inspection.

The composition used in step (a) is suitably in the form of a cream, gel or paste. It suitably has a consistency that enables it to be readily applied to and spread throughout the hair but then stays on the hair and does not run off.

The composition may be applied to the hair by any suitable means. Suitably it is brushed on to the hair to provide an even coverage. Suitably the composition used in step (a) is contacted with the hair using a liquor ratio of from 5:1 to 1 :1, preferably from 4:1 to 3:1, suitably about 2.5:1. By this we mean that 2.5 parts by weight of the composition is applied for each part by weight of hair used.

Step (b) of the method of the present invention involves contacting the hair with an acidic composition. In the method of the present invention steps (a) and (b) are carried out sequentially. There is no rinsing step between steps (a) and (b), that is the composition applied in step (a) is not rinsed from the hair with water prior to step (b). Thus step (b) of contacting the hair with an acidic composition involves applying the acidic composition to hair which remains in contact with the composition applied in step (a). Following step (a) excess composition may be brushed from or squeezed out of the hair but it is not removed by rinsing with water.

The acidic composition used in step (b) preferably has a pH of from 1 to 6.5, more preferably from 2 to 6, more preferably from 3 to 5, for example from 3 to 4 or from 3 to 3.5.

Suitable acids that may be included in the composition contacted with the hair in step (b) include organic and inorganic acids. Suitable organic acids include ascorbic acid, acetic acid, oxalic acid, lactic acid, formic acid, citric acid, glycolic acid, glucuronic acid, malic acid, mandelic acid and tartaric acid.

Suitable inorganic acids include hydrochloric acid, sulfuric acid, nitric acid, phosphorous acid, hypophosphorous acid and phosphoric acid.

The acidic composition applied in step (b) is preferably a reducing composition. Thus the composition preferably comprises a reducing agent. In some embodiments the reducing agent may be the same as the acid and the composition may comprise a reducing acid. Suitable reducing acids for use in the composition contacted with the hair in step (b) include ascorbic acid, oxalic acid, lactic acid, formic acid, citric acid glycolic acid, glucuronic acid, malic acid, mandelic acid and tartaric acid.

The skilled person will appreciate that under the conditions of pH used the acid may be present in the form of a salt. In some embodiments the acid may be incorporated into the composition in the form of a salt for ease of preparation.

One especially preferred reducing acid for use herein is ascorbic acid.

The organic reducing acid may be present in the composition applied to the hair in step (b) in an amount of from 0.1 to 10 wt%, suitably from 0.5 to 7.5 wt%, from 0.25 to 7.5 wt%, from 0.5 to 5 wt%, or from 0.5 to 1.5 wt%.

In preferred embodiments the organic reducing acid is present in the composition used in step (b) in an amount of from 0.1 to 25 wt%, preferably from 1 to 20 wt%, more preferably from 5 to 15 wt%, for example from 8 to 12 wt%.

The acidic composition applied to the hair in step (b) preferably further comprises a surfactant. Suitable surfactants will be known to the person skilled in art and include anionic, cationic, non-ionic and amphoteric surfactants. Preferably the composition includes an anionic and/or a cationic surfactant. Mixtures of surfactants may be present.

Suitable anionic surfactants for use herein include sulfate and sulfonate compounds including an alkyl or alkylene chain typically of 8 to 30 carbon atoms, preferably provided as an alkali metal or ammonium salt. Substituted ammonium salts may be used, for example tetraalkyl ammonium salts. Examples of suitable anionic surfactants include ammonium lauryl sulfate, alkyl ammonium lauryl sulfates, sodium lauryl sulfate, sodium laureth sulfate, sodium myreth sulfate and sodium dodecyl benzene sulfonate.

Suitable cationic surfactants for use herein include quaternary ammonium salts including one or more long alkyl or alkenyl chain typically of 8 to 30 carbon atoms, preferably provided as a chloride or bromide salt. Examples of suitable cationic surfactants include benzethonium trimethyl ammonium chloride, dimethyl dioctadecyl ammonium chloride, cetyl trimethyl ammonium chloride, stearyl trimethyl ammonium chloride and beheneth trimethyl ammonium chloride.

Suitable amphoteric surfactants include betaines and sultaines. Preferred betaines are alkyl betaines, for example cetyl betaine, lauryl betaine, stearyl betaine and behenyl betaine.

Other suitable surfactants for use herein will be known to the person skilled in the art.

Preferred surfactants for use in the composition contacted with the hair in step (b) include sodium dodecylsulphonate, sodium laureth sulphate, sodium lauryl sulphate, cetyl trimethyl ammonium chloride, stearyl trimethyl ammonium chloride, beheneth trimethyl ammonium chloride, tetra methyl ammonium lauryl sulfate, tetra ethyl ammonium lauryl sulphate, tetra methyl ammonium stearyl sulfate, tetraethyl stearyl ammonium sulfate, lauryl betaine, cetyl betaine and stearyl betaine.

In some embodiments the acidic composition contacted with the hair in step (b) comprises from 0.1 to 10 wt%, preferably from 0.5 to 7.5 wt% surfactants, for example from 1 to 5 wt%, preferably from 2 to 3 wt%.

In preferred embodiments the composition used in step (b) comprises from 5 to 30 wt% surfactants.

Suitably the composition comprises a mixture of surfactants. Preferably it comprises a mixture of anionic and amphoteric surfactants, for example a mixture of sulfates and betaines. The composition used in step (b) suitably comprise from 1 to 30 wt%, preferably from 5 to 20 wt% anionic surfactants and from 0.1 to 10 wt%, preferably from 1 to 5 wt% amphoteric surfactants.

As mentioned above the acidic composition contacted with the hair in step (b) preferably has a pH of from 2 to 6, more preferably from 3 to 5. The pH of the composition may be adjusted accordingly by inclusion of a suitable buffer. Suitable buffers will be known to the person skilled in the art and include for example 2-amino-2-methyl-1-proponol, sodium hydroxide and citric acid.

The composition used in step (b) may comprise one or more further ingredients, for example those typically found in shampoo compositions. Such ingredients include surfactants, thickeners, fragrances, colourants, preservatives, conditioning agents, cosolvents and chelating agents. Examples of such ingredients may be found on the INCI list.

The composition used in step (b) of the method of the present invention is preferably an aqueous composition. Suitably it comprises 50 to 99.9 wt% water. It may comprise from 70 to 99 wt%, suitably 90 to 98 wt% water. Preferably it comprises from 50 to 80 wt% water.

The composition used in step (b) is preferably in the form of a viscous liquid or gel. It suitably has a consistency similar to that of shampoo and is used in a similar manner.

In some preferred embodiments the composition used in step (b) is based on a standard shampoo formulation comprising a mixture of surfactants and optional excipients and which further comprises from 5 to 15 wt% of a reducing acid.

In step (b) the acidic composition is preferably liberally applied to the hair and spread thoroughly throughout the hair. This may be done manually using the fingers as one might spread shampoo throughout the hair.

Preferably after application of the acidic composition it is allowed to remain in contact with the hair for a period of at least 30 seconds, suitably at least 1 minute, preferably at least 2 minutes, preferably at least 3 minutes, for example at least 4 minutes. The composition may be allowed to remain in contact with the hair for up to 20 minutes, suitably up to 15 minutes, preferably up to 10 minutes, more preferably up to 8 minutes, for example up to 6 minutes.

Step (b) may suitably be carried out at ambient temperature. However it has been observed to be more efficient at higher temperatures.

Step (c) is carried out after step (b). Preferably following step (b) the hair is rinsed with hot water and then contacted with the oxidising composition used in step (c).

Step (c) of the method of the present invention involves contacting the hair with an oxidising composition. Any suitable oxidising composition may be used. Preferred oxidising compositions are those including hydrogen peroxide.

Preferably the composition contacted with the hair in step (c) comprises at least 0.1 wt% hydrogen peroxide, more preferably at least 0.5 wt%, suitably at least 0.75 wt%, for example at least 1 wt%. The composition contacted with the hair in step (c) of the method of the present invention may comprise up to 10 wt% hydrogen peroxide, suitably up to 7.5 wt%, preferably up to 5 wt%, suitably up to 4 wt%, for example up to 3 wt%.

In some especially preferred embodiments the composition contacted with the hair in step (c) comprises from 0.01 to 2.5 wt% hydrogen peroxide, preferably from 0.1 to 2 wt%, more preferably form 0.2 to 1 wt%, suitably from 0.3 to 0.7 wt%, for example about 0.5 wt%. It has been found that using levels of hydrogen peroxide between 0.25 and 0.75 wt% are effective but do not cause skin irritation to a user.

Preferably the composition contacted with the hair in step (c) comprises a surfactant. Suitable surfactants will be known to the person skilled in art and include anionic, cationic, non-ionic and amphoteric surfactants. Preferably the composition includes an anionic and/or a cationic surfactant. Mixtures of surfactants may be present.

Suitable anionic surfactants for use herein include sulfate and sulfonate compounds including an alkyl or alkylene chain typically of 8 to 30 carbon atoms, preferably provided as an alkali metal or ammonium salt. Substituted ammonium salts may be used, for example tetraalkyl ammonium salts. Examples of suitable anionic surfactants include ammonium lauryl sulfate, alkyl ammonium lauryl sulfates, sodium lauryl sulfate, sodium laureth sulfate, sodium myreth sulfate and sodium dodecyl benzene sulfonate.

Suitable cationic surfactants for use herein include quaternary ammonium salts including one or more long alkyl or alkenyl chain typically of 8 to 30 carbon atoms, preferably provided as a chloride or bromide salt. Examples of suitable cationic surfactants include benzethonium trimethyl ammonium chloride, dimethyl dioctadecyl ammonium chloride, cetyl trimethyl ammonium chloride, stearyl trimethyl ammonium chloride and beheneth trimethyl ammonium chloride.

Suitable amphoteric surfactants include betaines and sultaines. Preferred betaines are alkyl beanies, for example cetyl betaine, lauryl betaine, stearyl betaine and behenyl betaine.

Other suitable surfactants for use herein will be known to the person skilled in the art.

Preferred surfactants for use in the composition contacted with the hair in step (c) include sodium dodecylsulphonate, sodium laureth sulphate, sodium lauryl sulphate, cetyl trimethyl ammonium chloride, stearyl trimethyl ammonium chloride, beheneth trimethyl ammonium chloride, tetra methyl ammonium lauryl sulfate, tetra ethyl ammonium lauryl sulphate, tetra methyl ammonium stearyl sulfate, tetraethyl stearyl ammonium sulfate, lauryl betaine, cetyl betaine and stearyl betaine.

The composition used in step (c) preferably comprises from 5 to 30 wt% surfactants. Suitably the composition comprises a mixture of anionic and amphoteric surfactants, for example a mixture of sulfates and betaines.

The composition used in step (c) suitably comprises from 1 to 30 wt% preferably from 5 to 20 wt% anionic surfactants and from 0.1 to 10 wt% preferably from 1 to 5 wt% amphoteric surfactants.

The composition used in step (c) is preferably an aqueous based composition. Suitably it comprises from 50 to 99 wt% water, preferably from 60 to 90 wt%.

The composition contacted with the hair in step (c) preferably has a pH of from 2 to 6, more preferably from 3 to 5. The pH of the composition may be adjusted accordingly by inclusion of a suitable buffer. Suitable buffers will be known to the person skilled in the art and include for example citric acid, 2-amino-2-methyl-1-proponol and sodium hydroxide.

The composition used in step (c) may comprise one or more further ingredients, for example those typically found in shampoo compositions. Such ingredients include stabilisers, thickeners, fragrances, colourants, preservatives, cosolvents, conditioning agents and chelating agents. Examples of such ingredients may be found on the INCI list.

The composition used in step (c) is preferably in the form of a viscous liquid or gel. It suitably has a consistency similar to that of shampoo and is used in similar manner.

In step (c) the oxidising composition is preferably applied liberally to the hair and spread thoroughly throughout the hair. This may be done manually using the fingers as one might spread shampoo throughout the hair.

In step (c) the oxidising composition is suitably left in the hair for a period of 10 seconds to 10 minutes, preferably 20 seconds to 2 minutes, suitably up to 5 minutes and then rinsed from the hair thoroughly with hot water.

Hair treated according to the method of the present invention suitably has a colour that closely matches its colour prior to dyeing with an oxidative hair dye. In the case of a very strong colour or if the hair is very porous, the method may be repeated.

A particular advantage of the colour removal method of the present invention is that it only removes colour present in the hair due to oxidative dyeing of the hair. Unlike many colour removing compositions of the prior art which rely on bleach, the compositions used in the present invention do not destroy the natural melanin pigment present in the hair. Consequently the orange tone and significant damage to the hair which are often seen when using such prior art compositions are not observed when using the method of the present invention.

The colour of hair may be measured by measuring reflectance using a reflectance spectrophotometer. However hairdressers often make comparison with colour charts showing standard colours.

The method of the present invention may be used to remove colour from any hair that has been oxidatively dyed. The method may be carried out on hair that is on the human head. The method may be used to treat some or all of the hair, for example it may be used to provide highlights. Alternatively it may used to remove colour from hair that has been oxidatively dyed which is not on the human head, for example hair that is on a wig or a hair extension. The method in the invention may also be used to treat animal hair.

According to a second aspect of the present invention there is provided a kit for removal of colour from hair that has been oxidatively dyed, the kit comprising:
(i) a first composition comprising formamidine sulfinic acid;
(ii) a second acidic composition; and
(iii) a third oxidising composition.

Preferred features of the first composition are as defined in relation to the composition contacted with the hair in step (a) of the method of the first aspect. In some embodiments the kit may comprise two or more precursor compositions which on admixture form a composition comprising a sulfur-containing nucleophile or precursor thereof.

Preferred features of the second composition are as defined in relation to the composition contacted with the hair in step (b) of the method of the first aspect.

Preferred features of the third composition are as defined in relation to the composition contacted with the hair in step (c) of the method of the first aspect.

The kit may also comprise instructions for using the first, second and third compositions.

The present invention will now be further described with reference to the following non limiting examples.

### Example 1

In the examples the colour of the hair was assessed by reference to a chart showing standard reference samples in neutral colours. Level 1 on the chart is jet black hair and level 10 is a very light blonde.

A sample of level 6 light brown hair was optionally treated with a hair lightening mixture comprising the following ingredients:

| Part 1 | Part 2 |
|---|---|
| Commercial white persulphate bleach. | 9% hydrogen peroxide cream. |

The hair lightening mixture was prepared by mixing 3g Part 1 with 9g Part 2.

The mixture was brushed into hair and left at 40°C for 20 minutes using a liquor ratio of 2.5:1 (2.5g preparation mixture : 1 g hair tress).

It was then rinsed from the hair with cold or warm water and shampoo using a standard commercial shampoo. Then the hair was dried.

The resulting hair was bleached to level 9-10.

This hair was then coloured with an oxidative dye as follows:

| Part 1 | Part 2 |
|---|---|
| L'Oréal Majirel Number 3 oxidative crème. | 9% hydrogen peroxide cream. |

The hair colouring composition was prepared mixing 5g of Part 1 with 7.5g Part 2.

This mixture was brushed into hair and left at 40°C for 30 minutes using a liquor ratio of 2.5:1 (2.5g preparation mixture : 1 g hair tress).

The hair was then rinsed with cold or warm water and shampoo using a standard commercial shampoo. Then the hair was dried.

The resulting hair was a dark brown colour of approximately level 3 darkness.

The hair then treated with a composition according to step (a) of the method of the present invention as follows:

| Part 1 | | Part 2 | |
|---|---|---|---|
| 5.555% | Urea | 50% | Magnesium acetate, tetrahydrate. |
| 1.11% | Thioglycolic acid | 50% | Formamidine sulfinic acid |
| 1.5% | Aminomethyl propanol | | |
| 2.22% | Hydroxyethylcellulose | | |
| 89.615% | Water | | |

Percentage values in the table above denote the weight of a given component expressed as a percentage of the total weight of the composition of the part in which it is present.

The composition was prepared by mixing 9g of Part 1 with 1 g Part 2. Part 1 is in the form of a gel and part 2 is in the form of two powder-containing sachets (to keep the components separate).

This mixture was brushed into the hair and left at 40°C for 30 minutes using a liquor ratio of 2.5:1 (2.5g preparation mixture : 1 g hair tress).

At this point the hair tress has the appearance of a level 9-10 blonde, the oxidative dye having been decolourised.

Excess composition was manually squeezed out of the hair without washing/rinsing.

The hair was then treated with a composition according to step (b) of the method of the present invention comprising the following active ingredients:

| |
|---|
| 1% Ascorbic Acid |
| 2.5 % Sodium dodecylbenzene sulphonate |
| Buffered to pH 3.0-3.5 |

The composition was liberally applied and left for 5 minutes, before rinsing with hot water.

According to step (c) of the method of the present invention the hair was then treated with a composition containing 2% hydrogen peroxide. The composition was applied to the hair in the manner of a shampoo and then rinsed away with water before drying the hair.

The resulting hair had the majority of the oxidative dye removed and was now a neutral shade, approximately level 7-8.

A second treatment according to steps (a), (b) and (c) lightens the hair further, resulting in hair of level 8-9.

There is little or no damage to the hair following multiple treatments.

The resulting shade is dependent on the underlying colour of the hair pre-oxidative dyeing. Lighter dyeings are more effectively returned to their original colour.

The resulting colour of hair after treatment according to the present invention is neutral to slightly ashy. This is in contrast to traditional methods of removing oxidative dyes by bleaching, which leaves orange/red tones, together with damaged hair.

### Example 2

Light brown hair was bleached to level 9-10 using the same method described in example 1.

This hair was then coloured with an oxidative dye as follows:

| **Part 1** | **Part 2** |
|---|---|
| L'Oréal Majirel Number 3 (Permanent Oxidative Hair Colour) | Hydrogen Peroxide (9%) cream. |

The hair colouring composition was prepared mixing 5g of Part 1 with 7.5g Part 2.

This mixture was brushed into hair and left at 40°C for 30 minutes using a liquor ratio of 2.5:1 (2.5g preparation mixture : 1 g hair tress).

The hair was then rinsed with cold or warm water and shampoo using a standard commercial shampoo. Then the hair was dried.

The resulting hair was a dark brown colour of approximately a level 3 darkness.

The hair then treated with a composition according to step (a) of the method of the present invention as follows:

| **Part 1** | | **Part 2** | |
|---|---|---|---|
| 5.55% | Urea | 50% | Magnesium acetate, tetrahydrate. |
| 1.11% | Thioglycolic acid | 50% | Formamidine sulfinic acid |
| 1.50% | Aminomethyl propanol | | |
| 2.22% | Hydroxyethylcellulose | | |
| 89.62% | Water | | |

Percentage values in the table above denote the weight of a given component expressed as a percentage of the total weight of the composition of the part in which it is present.

The composition was prepared by mixing 9g of Part 1 with 1 g Part 2. Part 1 is in the form of a gel and part 2 is in the form of two powder-containing sachets (to keep the components separate).

This mixture was brushed into the hair and left at 40°C for 30 minutes using a liquor ratio of 2.5:1 (2.5g preparation mixture : 1 g hair tress).

At this point the hair tress has the appearance of a level 9-10 blonde, the oxidative dye having been decolourised.

Excess composition was manually squeezed out of the hair without washing/rinsing.

The hair was then treated with a composition according to step (b) of the method of the present invention.

The composition was an aqueous based mixture comprising a mixture of an anionic sulphate surfactant and an amphoteric betaine surfactant (total approximately 30 wt% surfactants), along with 10 wt% ascorbic acid. The composition also contained small amounts of cosolvents, stabilisers and preservatives.

The mixture had a pH of 3.0-3.5.

The composition was liberally applied and left for 5 minutes, before rinsing with warm water.

According to step (c) of the method of the present invention the hair was then treated with a composition containing 0.5 wt% hydrogen peroxide along with a mixture of an anionic sulphate surfactant and an amphoteric betaine surfactant (total approximately 30 wt% surfactants). Also present in the composition were a buffer, cosolvents, stabilisers and preservatives.

The composition had a pH of 4.

The composition was applied to the hair in the manner of a shampoo and then rinsed away with water before drying the hair.

The resulting hair had the majority of the oxidative dye removed and was now a neutral shade, approximately level 7-8.

A second treatment according to steps (a), (b) and (c) lightened the hair further, resulting in hair of level 8-9.

Little or no damage to the hair was observed following multiple treatments.

The resulting shade is dependent on the underlying colour of the hair pre-oxidative dyeing. Lighter dyeings are more effectively returned to their original colour.

The resulting colour of hair after treatment according to the present invention is neutral to slightly ashy. This is in contrast to traditional methods of removing oxidative dyes by bleaching, which leaves orange/red tones, together with damaged hair.

### Example 3

A sample of light brown hair was bleached to level 9-10 using the same method described in example 1.

This hair was then coloured with an oxidative dye as follows:

| **Part 1** | **Part 2** |
|---|---|
| L'Oréal Majirel Number 3 (Permanent Oxidative Hair Colour) | Hydrogen Peroxide (9%) cream. |

The hair colouring composition was prepared mixing 5g of Part 1 with 7.5g Part 2.

The hair was coloured using the same conditions described in example 2.

The resulting hair was a black colour of approximately level 3 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 8-9, after a single treatment.

### Example 4

A sample of light brown hair was bleached to level 9-10 using the same method described in example 1.

This hair was then coloured with an oxidative dye as follows:

| **Part 1** | **Part 2** |
|---|---|
| L'Oréal Majirel Number 1 (Permanent Oxidative Hair Colour) | Hydrogen Peroxide (9%) cream. |

The hair was coloured using the same conditions described in example 2.

The resulting hair was a black colour of approximately level 1 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 8-9, after a single treatment.

### Example 5

A sample of light brown hair was bleached to level 9-10 using the same method described in example 1.

This hair was then coloured with an oxidative dye as follows:

| **Part 1** | **Part 2** |
|---|---|
| L'Oréal Majirel Number 1 (Permanent Oxidative Hair Colour) | Hydrogen Peroxide (9%) cream. |

The hair was coloured using the same conditions described in example 2.

The resulting hair was a black colour of approximately level 1 darkness.

The hair then treated with a composition according to step (a) of the method of the present invention as follows:

| **Part 1** | | **Part 2** | |
|---|---|---|---|
| 33.33% | Urea | 50% | Magnesium acetate, tetrahydrate. |
| 1.11% | Thioglycolic acid | 50% | Formamidine sulfinic acid |
| 5.55% | Polysorbate 20 | | |
| 1.50% | Aminomethyl propanol | | |
| 2.22% | Hydroxyethylcellulose | | |
| 56.29% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 8-9, after a single treatment.

### Example 6

A sample of light brown hair was bleached to level 9-10 using the same method described in example 1.

This hair was then coloured with an oxidative dye as follows:

| **Part 1** | **Part 2** |
|---|---|
| L'Oréal Majicontrast Magenta Red (Permanent Oxidative Hair Colour) | Hydrogen Peroxide (9%) cream. |

The hair was coloured using the same conditions described in example 2.

The resulting hair was a bright red of approximately level 8 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had 100% of the oxidative dye removed and was now a neutral shade, approximately level 10, after a single treatment.

### Example 7

A sample of light brown hair was bleached to level 9-10 using the same method described in example 1.

This hair was then coloured with an oxidative dye: Schwarzkopf Live Colour XXL 50 Hot Cinnamon home kit.

The hair was coloured using the same conditions described in example 2.

The resulting hair was a reddish brown colour of approximately level 5 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 8-9, after a single treatment.

### Example 8

A sample of light brown hair was bleached to level 9-10 using the same method described in example 1.

This hair was then coloured with an oxidative dye: Clairol nice'n easy 6R light auburn home kit.

The hair was coloured using the same conditions described in example 2.

The resulting hair was a copper brown colour of approximately level 6 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 9, after a single treatment.

### Example 9

A sample of light brown hair (Level 8) was coloured with an oxidative dye as follows:

| **Part 1** | **Part 2** |
|---|---|
| L'Oréal Majirel Number 3 (Permanent Oxidative Hair Colour) | Hydrogen Peroxide (9%) cream. |

The hair was coloured using the same conditions described in example 2.

The resulting hair was a dark brown colour of approximately level 3 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 7 to 8, after a single treatment.

### Example 10

A sample of medium brown hair (Level 6) was coloured with an oxidative dye as follows:

| **Part 1** | **Part 2** |
|---|---|
| L'Oréal Majirel Number 3 (Permanent Oxidative Hair Colour) | Hydrogen Peroxide (9%) cream. |

The hair was coloured using the same conditions described in example 2.

The resulting hair was a dark brown colour of approximately level 3 darkness.

The hair then treated with a composition according to step (a) of the method of the present invention as follows:

| **Part 1** | | **Part 2** | |
|---|---|---|---|
| 22.22% | Urea | 50% | Magnesium acetate, tetrahydrate. |
| 1.11% | Thioglycolic acid | 50% | Formamidine sulfinic acid |
| 5.55% | Polysorbate 80 | | |
| 1.50% | Aminomethyl propanol | | |
| 2.22% | Hydroxyethylcellulose | | |
| 67.40% | Water | | |

The application method of this composition to the hair was as described in example 2.

After the treatment time, excess composition was manually squeezed out of the hair without washing/rinsing.

The subsequent applications of compositions according to steps (b) & (c) of the method of the present invention were also as described in example 2.

The resulting hair had the majority of the oxidative dye removed and was now a neutral shade, approximately level 6 to 7, after a single treatment.

## Claims

1. A method of removing colour from hair that has been oxidatively dyed, the method comprising the steps of:
(a) contacting the hair with a composition comprising formamidine sulfinic acid;
(b) contacting the hair with an acidic composition; and
(c) contacting the hair with an oxidising composition;
wherein there is no rinsing step between step (a) and step (b).

2. A method according to any preceding claim wherein the composition used in step (a) further comprises thioglycolic acid.

3. A method according to claim 1 or claim 2 wherein the composition used in step (a) comprises urea.

4. A method according to any preceding claim wherein the composition used in step (b) is a reducing composition.

5. A method according to any preceding claim wherein the hair is rinsed following step (b) with hot water and then contacted with the oxidising composition used in step (c).

6. A method according to any preceding claim wherein the composition used in step (c) comprises hydrogen peroxide.

7. A kit for removal of colour from hair that has been oxidatively dyed, the kit comprising:
(i) a first composition comprising sulfamidine sulfinic acid;
(ii) a second acidic composition; and
(iii) a third oxidising composition.

## Patentansprüche

1. Verfahren zur Entfernung von Farbe aus oxidativ gefärbtem Haar, wobei das Verfahren die folgenden Schritte umfasst:
(a) Inkontaktbringen des Haars mit einer Zusammensetzung, die Formamidinsulfinsäure umfasst;
(b) Inkontaktbringen des Haars mit einer sauren Zusammensetzung; und
(c) Inkontaktbringen des Haars mit einer oxidierenden Zusammensetzung;
wobei zwischen Schritt (a) und Schritt (b) kein Ausspülschritt erfolgt.

2. Verfahren nach einem der vorhergehenden Ansprüche, wobei die in Schritt (a) verwendete Zusammensetzung ferner Thioglykolsäure umfasst.

3. Verfahren nach Anspruch 1 oder Anspruch 2, wobei die in Schritt (a) verwendete Zusammensetzung Harnstoff umfasst.

4. Verfahren nach einem der vorhergehenden Ansprüche, wobei die in Schritt (b) verwendete Zusammensetzung eine reduzierende Zusammensetzung ist.

5. Verfahren nach einem der vorhergehenden Ansprüche, wobei das Haar nach Schritt (b) mit warmem Wasser ausgespült und dann mit der in Schritt (c) verwendeten oxidierenden Zusammensetzung in Kontakt gebracht wird.

6. Verfahren nach einem der vorhergehenden Ansprüche, wobei die in Schritt (c) verwendete Zusammensetzung Wasserstoffperoxid umfasst.

7. Kit zur Entfernung von Farbe aus oxidativ gefärbtem Haar, wobei das Kit umfasst:
(i) eine erste Zusammensetzung, die Sulfamidinsulfinsäure umfasst;
(ii) eine zweite saure Zusammensetzung und
(iii) eine dritte oxidierende Zusammensetzung.

## Revendications

1. Procédé d'élimination d'une couleur de cheveux ayant été teintés de façon oxydante, le procédé comprenant les étapes suivantes :
(a) mise en contact des cheveux avec une composition comprenant de l'acide formamidinesulfinique ;
(b) mise en contact des cheveux avec une composition acide ; et
(c) mise en contact des cheveux avec une composition oxydante ;
où il n'y a pas d'étape de rinçage entre l'étape (a) et l'étape (b).

2. Procédé selon l'une quelconque des revendications précédentes, où la composition utilisée dans l'étape (a) comprend en outre de l'acide thioglycolique.

3. Procédé selon la revendication 1 ou la revendication 2, où la composition utilisée dans l'étape (a) comprend de l'urée.

4. Procédé selon l'une quelconque des revendications précédentes, où la composition utilisée dans l'étape (b) est une composition réductrice.

5. Procédé selon l'une quelconque des revendications précédentes, où les cheveux sont rincés après l'étape (b) sans eau chaude avant d'être mis en contact avec la composition oxydante utilisée dans l'étape (c).

6. Procédé selon l'une quelconque des revendications précédentes, où la composition utilisée dans l'étape (c) comprend du peroxyde d'hydrogène.

7. Kit d'élimination d'une couleur de cheveux ayant été teintés de façon oxydante, le kit comprenant :
(i) une première composition comprenant de l'acide sulfamidinesulfinique ;
(ii) une deuxième composition acide ; et
(iii) une troisième composition oxydante.
